# EUROPEAN PATENT SPECIFICATION

(11) **EP 1 441 648 B1**
(45) Date of publication and mention of the grant of the patent: **14.12.2011**
(21) Application number: 01985170.8
(22) Date of filing: 20.10.2001
(51) Int. Cl.: A61B 17/00

(54) **WOUND RETRACTION APPARATUS**
WUNDRETRAKTIONSVORRICHTUNG
APPAREIL D'ECARTEMENT DE LESIONS

(43) Date of publication of application: 04.08.2004
(60) Divisional application: 10184608.7
(73) Proprietor: APPLIED MEDICAL RESOURCES CORPORATION, Rancho Santa Margarita, CA 92688 (US)
(72) Inventor: EWERS, Richard, C., Fullerton, CA 92833 (US); BRUSTAD, John, R., Dana Point, CA 92629 (US); PINGLETON, Edward, D., Laguna Niguel, CA 92677 (US); HILAL, Nabil, Laguna Niguel, CA 92677 (US); ADLPARVAR, Payam, Lake Forest, CA 92630 (US); TAYLOR, Scott, Mission Viejo, CA 92692 (US); DULAK, Gary, R., Newport Beach, CA 92663 (US); DUNN, Michael, J., Santa Ana, CA 92706 (US); MORALES, Norman, San Jose, CA 95126 (US); HART, Charles, C., Summerville, SC 29483-8949 (US); BOWES, Robert, R., II, Laguna Niguel, CA 92677 (US)
(74) Representative: Fitchett, Stuart Paul
(86) International application number: PCT/US2001/050742
(87) International publication number: WO 2003/061480

(56) References cited:
- EP-A- 0 849 517
- WO-A-00/32116
- WO-A-01/26559
- US-A- 5 514 133
- US-A- 5 906 577
- US-A- 6 090 043
- US-A- 6 162 172
- US-B1- 6 254 533
- US-B1- 6 254 534

## Description

### Background of the Invention

### Field of Invention

This invention relates generally to wound retraction and more specifically to wound retraction in a laparoscopic surgical procedure.

### Discussion of the Related Art

During laparoscopic surgery, it is desirable to inflate the abdominal cavity in order to increase the volume of the working space. This is accomplished with an insufflation gas which must be maintained at a pressure sufficient to inflate the abdomen. Maintaining the pressure of the insufflation gas is difficult when it is also desirable to insert instrumentation through the abdominal wall. If the surgeon is interested in inserting his or her hand in a hand-assisted laparoscopic procedure, the maintenance of insufflation pressure is even more difficult. Currently, several devices exist that accomplish this surgical need although they suffer from drawbacks such as difficult placement and cumbersome use. Thus, it is desirable that the wound be retracted, protected, and fixed while maintaining an insufflation seal.

Wound retraction in accordance with the present invention allows the surgeon to easily locate a retractor and to provide a solid base for an instrument or hand seal. This retractor removes the tissue pressure from the wrist during hand-assisted laparoscopic surgery. It can also protect the tissue at the wound site, for example, from abrasion, bacteria or other contaminants organs, such as donor kidneys to be removed with minimal risk or damage. The retractor also opens the wound providing greater access to the operative site for instruments, such as the hand of the surgeon.

### Description of the Drawings

Figures 1 to 3 are perspective views illustrating the basic concept of retracting a wound using a retractor;
Figure 4 is a perspective view of an embodiment of the invention including an elastomeric sleeve and multiple retention elements secured onto hook features;
Figure 5 having a sheath attached to an outer ring with clamps;
Figure 6 is a perspective view of a further embodiment of the invention including multiple ladders with rungs providing a sheath with desired tension;
Figure 7A is a perspective view of a further embodiment of the invention including retention elements in the form of zip ties with detents; and
Figure 7B is an enlarged perspective view of a detent adapted for use in the embodiment of Figure 7A.

### Description of Preferred Embodiments and Best Mode of the Invention

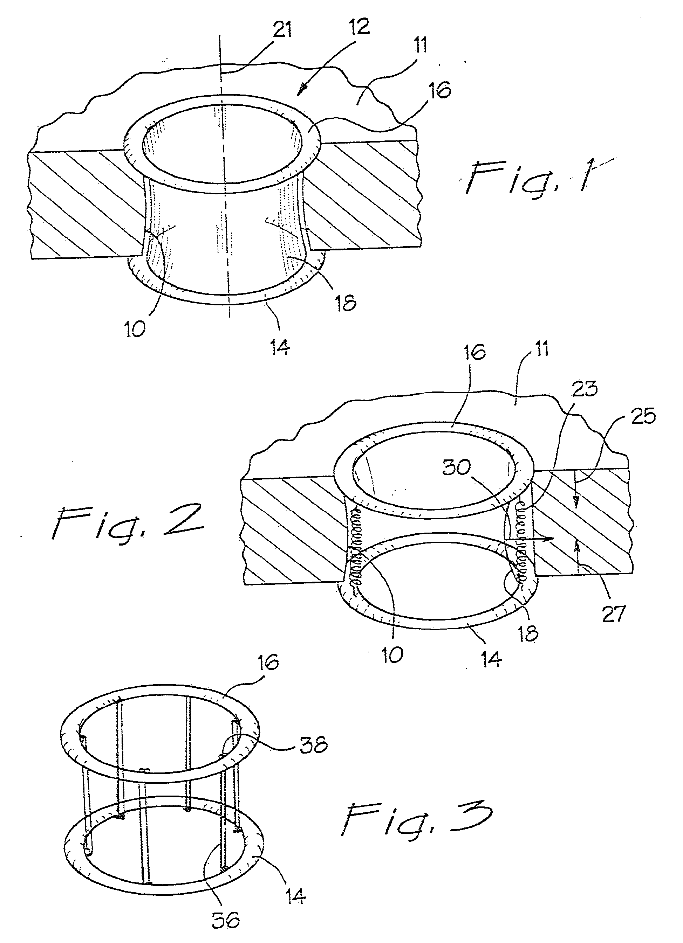

The basic concept of retracting and protecting a wound site is illustrated in the prospective view of Figure 1 wherein a wound 10 is formed in an abdominal wall 11. In this embodiment, a retractor 12 uses two rings 14 and 16 which are fixed to an elastic sheath 18. The sheath 18 has a generally cylindrical configuration and is disposed along an axis 21. The rings 14 and 16 are disposed in respective planes which extend radially of the axis 21.

The sheath 18 has elastomeric properties, but in its natural, upstretched state the two rings 14 and 16 are separate by a natural distance. The lower ring 14 is placed interiorly of the abdominal wall 11 and the upper ring 14 is stretched beyond the natural distance away from the lower ring. Once the elastic sheath 18 has been stretched to a distance greater than the abdominal wall thickness, the upper ring 16 is placed on the surface of the skin.

Since the diameters of the rings 14, 16 are greater than that desired for the wound site 10, they will have sufficient footing to maintain this tension between the two rings 14, 16. This tension is created by the elastic material that has been stretched and retained at a distance greater than the natural distance. It will be appreciated that in many embodiments, the sheath 18 can be formed of a non-elastic sheathing material. In a similar manner, the rings 14 and 16 may be provided with a rigid configuration or alternatively may be formed of an elastomeric material.

Figure 2 shows a simple schematic of the ring dynamics illustrated in Figure 1, with the retractor 12 operatively disposed across the abdominal wall 11. The elastic sheet sheath 18 acts as a circumferential spring 23 around the wound site 10 that evenly distributes the tension between the two rings 14 and 16, as represented by arrows 25 and 27. In addition, the elastic sheeting provides a radial retraction force 30 around the wound to enlarge the wound site 10 in order to facilitate the passage of instruments, such as the hand of the surgeon.

The amount of tension force between the two rings 14 and 16 can be controlled by the elastomeric proportion of the elastic sheath 18. In order to accommodate a larger range of abdominal wall thicknesses, a material with a higher elasticity can be chosen to allow for greater stretch.

Figure 3 illustrates the "sandwiching" of the abdominal wall between the two rings 14, 16 using elastic tensioners, such as rubber bands 36. The use of elastic tensioners allows for a greater range of wall thicknesses because the tensions can be selected at the time of application. For instance, the user would make an incision and measure the actual abdominal wall thickness. He would then choose the appropriate rubber bands providing the desired tensions for the given application. In this case the rubber bands 36 could be secured by hooks 38 provided on one or both of the rings 14 and 16. This gives the user more versatility with the device.

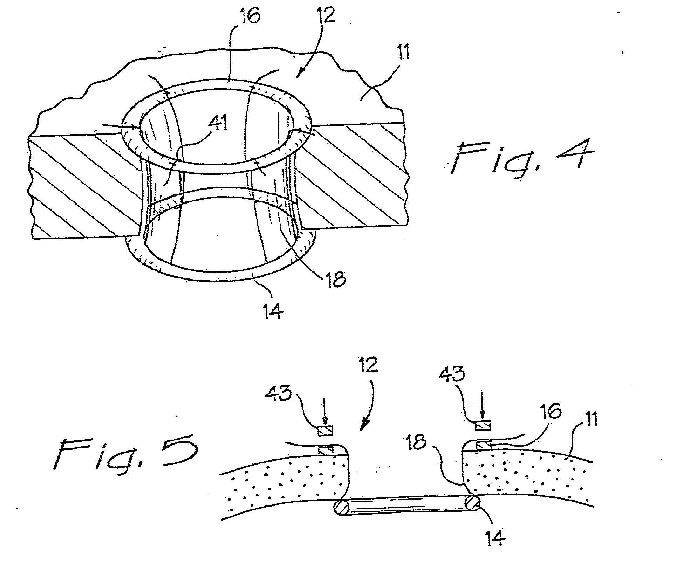

Figure 4 is an embodiment in accordance with the present invention which provides even further versatility. This design is not limited by the fixed distance between the rings 14, 16 of the retractor 12. Instead, the inner ring 14 will be fixed to the sheath 18, and the upper ring 16 will comprise a separate assembly. Initially, the inner ring 14 is placed into the peritoneum cavity. Then the elastic material is pulled taut and secured onto hook features 41 located on the outer ring 16. This allows the user to fix the retractor 12 to an abdominal wall of any size.

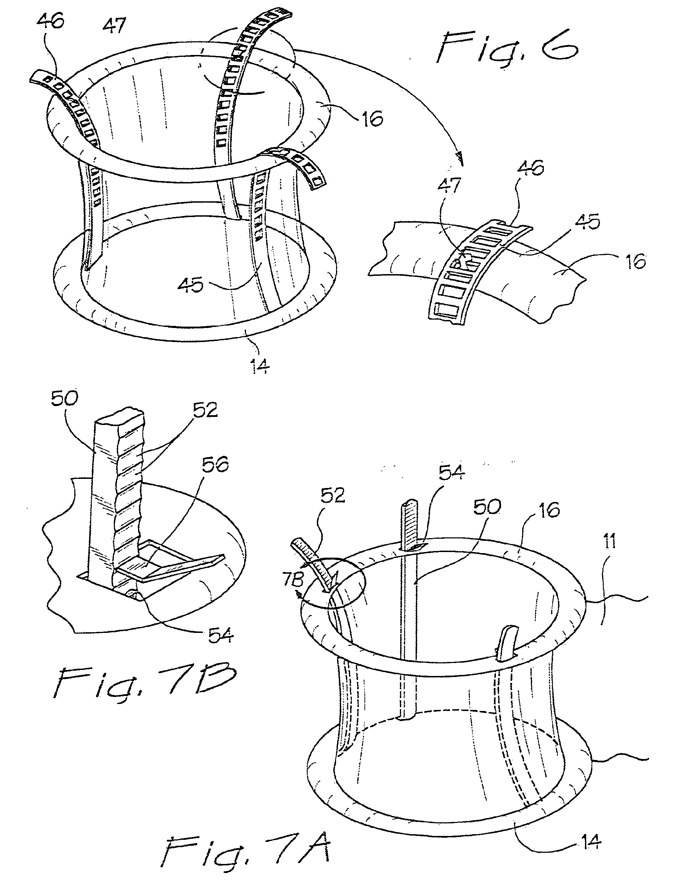

Figure 5 shows an embodiment not in accordance with the present invention wherein the sheath 18 is attached to the outer ring 16 using clamps 43. Figure 6 illustrates another embodiment in accordance with the present invention wherein the sheath 18 is attached to the outer ring 16 using ladders 45 with rungs 46, that may be connected to the internal surface of the sheath 18. Once the preferred tension of the sheath 18 has been attained, the user simply hooks an appropriate rung 46 onto an associated hook 47 located on the outer ring 16.

Figure 7A shows a structure which sandwiches the abdominal wall 11 between the two rings 14,16 using zip ties 50. After the inner ring 14 has been placed, the ties 50 can be pulled tight through associated holes 54 to fix the rings 14, 16 relative to the abdominal wall 11. As illustrated in Figure 7B, the zip ties 50 maybe formed with a plurality of grooves 52 which extend traverse or perpendicular to the length of the tie 50. In operation, the tie 50 is threaded through the hole 54 in the upper ring 16 where one of the grooves 52 is engaged by a tongue 56 which prevents movement of the tie 50 back through the hole 54.

It will be understood that many other modifications can be made to the various disclosed embodiments without departing from the scope of the claims. For example, various sizes of the surgical device are contemplated as well as various types of constructions and materials. It will also be apparent that many modifications can be made to the configuration of parts as well as their interaction. For these reasons, the above description should not be construed as limiting the invention, but should be interpreted as merely exemplary of preferred embodiments. Those skilled in the art will envision other modifications within the scope of the present invention as defined by the following claims.

## Claims

1. A surgical wound retractor (12) adapted to dilate a wound stretchable to a desired diameter, the retractor comprising:
a first ring (14) having a diameter greater than the desired diameter of the wound and being adapted for this disposition interiorly of the wound;
a second ring (16) having a diameter greater than the desired diameter of the wound and being adapted for disposition exteriorly of the wound;
a sheath (18) disposed in a cylindrical form between the first ring (14) and the second ring (16) wherein the sheath (18) is adapted for disposition through the wound to exert a radial retraction force on the wound, the retraction force being dependent on a distance separating the first ring (14) and the second ring (16); and
a plurality of retention elements (46) disposed in a generally cylindrical relationship to each other inside the sheath and extending between the first ring (14) and the second ring (16),
wherein at least one of each of the first ring (14) and the second ring (16) comprises a plurality of retainers (47) for engaging the plurality of retention elements (46).
**characterized in tha**tthe sheath is fixed to the first ring (14) and the second ring (16).

2. The surgical wound retractor recited in Claim 1, wherein:
the plurality of retention elements includes a plurality of the elastomeric bands; and
the retraction force is dependent on the elastomeric properties of the elastomeric bands.

3. The surgical wound retractor of Claim 2, wherein
each of the elastomeric bands is adapted for retention by an associated retainers on at least one ring.

4. The surgical wound retractor recited in any of Claims 1 to 3, wherein the retainers comprise a plurality of hooks.

5. The surgical wound retractor recited in any of Claims 2 to 4, wherein the elastomeric bands are selected from a group of elastomeric bands each, having different elastomeric properties.

6. The surgical wound retractor of any of Claims 1 to 5, wherein
the retention elements are adapted for disposition through the wound to exert a radial retraction force on the wound of sufficient magnitude to dilate the wound to the desired diameter, wherein the retraction force is dependent on the distance separating the first ring and the second ring;
the retention elements each comprise a distal end and a proximal end, wherein each distal end of the retention element is coupled to the first ring each retention element comprises a plurality of engagement sites disposed between the proximal end and the distal end, wherein each engagement site is associated with a different retraction force; and
the second ring comprises the plurality of retainers, wherein each retainer is dimensioned and configured for engaging a selected one of the engagement sites to provide an associated radial retraction force.

7. The surgical wound retractor recited in Claim 6, wherein the engagement sites of the retention elements include a plurality of ladder rungs, wherein each ladder rung is associated with a different retraction force.

8. The surgical wound retractor recited in Claim 6, wherein:
the engagement sites include a plurality of grooves, wherein each groove is associated with a different retraction force; and
each retainer of the second ring includes a lever bias to engage a selected one of the grooves to provide the associated retraction force.

9. The surgical wound retractor recited in Claim 6, wherein:
the engagement sites include a plurality of enlargements, wherein each enlargement is associated with a different retraction force; and
each retainer of the second ring has properties for engaging a selected one of the enlargements to provide the associated retraction force.

## Patentansprüche

1. Ein chirurgischer Wundretraktor (12), der dazu angepasst ist, eine zu einem gewünschten Durchmesser dehnbare Wunde zu erweitern, wobei der Retraktor umfasst:
einen ersten Ring (14) mit einem Durchmesser, der größer ist als der gewünschte Durchmesser der Wunde, und der zu dieser Anordnung innerhalb der Wunde angepasst ist;
einen zweiten Ring (16) mit einem Durchmesser, der größer ist als der gewünschte Durchmesser der Wunde, und der zur Anordnung außerhalb der Wunde angepasst ist;
eine Hülle (18), die in einer allgemein zylindrischen Form zwischen dem ersten Ring (14) und dem zweiten Ring (16) angeordnet ist, wobei die Hülle (18) zur Anordnung durch die Wunde angepasst ist, um eine radiale Rückhaltekraft auf die Wunde auszuüben, wobei die Rückhaltekraft von einem Abstand abhängig ist, der den ersten Ring (14) und den zweiten Ring (16) trennt;
eine Vielzahl von Halteelementen (46), die in einer allgemein zylindrischen Beziehung zueinander innerhalb der Hülle angeordnet sind und sich zwischen dem ersten Ring (14) und dem zweiten Ring (16) erstrecken,
wobei mindestens einer von dem ersten Ring (14) und dem zweiten Ring (16) eine Vielzahl von Halterungen (47) umfasst, um eine Vielzahl von Halteelementen (46) in Eingriff zu nehmen, **gekennzeichnet dadurch, dass** die Hülle an dem ersten Ring (14) und dem zweiten Ring (16) befestigt ist.

2. Der in Anspruch 1 genannte chirurgische Wundretraktor, wobei:
die Vielzahl von Halteelementen eine Vielzahl von elastomeren Bändern enthält; und
die Rückhaltekraft von den elastomeren Eigenschaften der elastomeren Bänder abhängig ist.

3. Der chirurgische Wundretraktor von Anspruch 2, wobei
jedes der elastomeren Bänder zur Retention durch eine damit verbundene Halterung an mindestens einem Ring angepasst ist.

4. Der in einem beliebigen der Ansprüche 1 bis 3 genannte chirurgische Wundretraktor, wobei die Halterungen eine Vielzahl von Haken umfassen.

5. Der in einem beliebigen der Ansprüche 2 bis 4 genannte chirurgische Wundretraktor, wobei die elastomeren Bänder jeweils aus einer Gruppe von elastomeren Bändern ausgewählt sind, die unterschiedliche elastomere Eigenschaften aufweisen.

6. Der chirurgische Wundretraktor gemäß einem beliebigen der Ansprüche 1 bis 5, wobei
die Halteelemente zur Anordnung durch die Wunde angepasst sind, um eine radiale Rückhaltekraft auf die Wunde auszuüben, die ausreichend groß ist, um die Wunde zu dem gewünschten Durchmesser zu erweitern, wobei die Rückhaltekraft von dem Abstand abhängig ist, der den ersten Ring und den zweiten Ring trennt;
die Halteelemente jeweils ein distales Ende und ein proximales Ende umfassen, wobei jedes distale Ende des Halteelements an den ersten Ring gekoppelt ist, jedes Halteelement eine Vielzahl von Eingriffsstellen umfasst, die zwischen dem proximalen Ende und dem distalen Ende angeordnet sind, wobei jede Eingriffsstelle mit einer unterschiedlichen Rückhaltekraft verbunden ist; und
der zweite Ring eine Vielzahl von Halterungen umfasst, wobei jede Halterung zum Eingriff mit einer ausgewählten der Eingriffsstellen dimensioniert und konfiguriert ist, um eine damit verbundene radiale Rückhaltekraft bereitzustellen.

7. Der in Anspruch 6 genannte chirurgische Wundretraktor, wobei die Eingriffsstellen der Halteelemente eine Vielzahl von Leitersprossen enthalten, wobei jede Leitersprosse mit einer unterschiedlichen Rückhaltekraft verbunden ist.

8. Der in Anspruch 6 genannte chirurgische Wundretraktor, wobei:
die Eingriffsstellen eine Vielzahl von Rillen enthalten, wobei jede Rille mit einer unterschiedlichen Rückhaltekraft verbunden ist; und
jede Halterung des zweiten Rings eine Hebelneigung enthält, um eine ausgewählte der Rillen in Eingriff zu nehmen, um die damit verbundene Rückhaltekraft bereitzustellen.

9. Der in Anspruch 6 genannte chirurgische Wundretraktor, wobei:
die Eingriffsstellen eine Vielzahl von Vergrößerungen enthalten, wobei jede Vergrößerung mit einer unterschiedlichen Rückhaltekraft verbunden ist; und
jede Halterung des zweiten Rings Eigenschaften zum Eingriff einer ausgewählten der Vergrößerungen aufweist, um die damit verbundene Rückhaltekraft bereitzustellen.

## Revendications

1. Écarteur de plaies chirurgicales (12) adapté de manière à dilater une plaie étirable jusqu'à obtention d'un diamètre désiré, l'écarteur comprenant :
un premier anneau (14) ayant un diamètre supérieur au diamètre désiré de la plaie et étant adapté pour être disposé intérieurement à la plaie ;
un second anneau (16) ayant un diamètre supérieur au diamètre désiré de la plaie et étant adapté pour être disposé extérieurement à la plaie ;
une gaine (18) disposée en une forme généralement cylindrique entre le premier anneau (14) et le second anneau (16), dans lequel la gaine (18) est adaptée pour être disposée au travers de la plaie en vue d'exercer une force de rétraction radiale sur la plaie, la force de rétraction étant dépendante d'une distance séparant le premier anneau (14) et le second anneau (16) ;
une pluralité d'éléments de retenue (46) disposés en un rapport généralement cylindrique l'un par rapport à l'autre à l'intérieur de la gaine et s'étendant entre le premier anneau (14) et le second anneau (16),
dans lequel au moins un de chacun du premier anneau (14) et du second anneau (16) comprend une pluralité de barres de retenue (47) pour engager la pluralité d'éléments de retenue (46), **caractérisé en ce que** la gaine est fixée au premier anneau (14) et au second anneau (16).

2. Écarteur de plaies chirurgicales selon la revendication 1, dans lequel :
la pluralité d'éléments de retenue inclut une pluralité de barres élastomères ; et la force de rétraction est dépendante des propriétés élastomères des barres élastomères.

3. Écarteur de plaies chirurgicales selon la revendication 2, dans lequel :
chacune des barres élastomères est adaptée pour la retenue par une barre de retenue associée sur au moins un anneau.

4. Écarteur de plaies chirurgicales selon l'une quelconque des revendications 1 à 3, dans lequel les barres de retenue sont munies d'une pluralité de crochets.

5. Écarteur de plaies chirurgicales selon l'une quelconque des revendications 2 à 4, dans lequel les barres élastomères sont sélectionnées parmi un groupe constitué par des barres élastomères, chacune ayant des propriétés élastomères différentes.

6. Écarteur de plaies chirurgicales selon l'une quelconque des revendications 1 à 5, dans lequel :
les éléments de retenue sont adaptés pour être disposés au travers de la plaie pour exercer une force de rétraction radiale sur la plaie, d'une ampleur suffisante pour dilater la plaie jusqu'à obtention du diamètre désiré, dans lequel la force de rétraction est dépendante de la distance séparant le premier anneau et le second anneau ;
les éléments de retenue comprennent chacun une extrémité distale et une extrémité proximale, dans lesquels chaque extrémité distale de l'élément de retenue est accouplée au premier anneau, chaque élément de retenue comprend une pluralité de sites d'engagement disposés entre l'extrémité proximale et l'extrémité distale, dans lesquels chaque site d'engagement est associé à une force de rétraction différente ; et
le second anneau comprend la pluralité de barres de retenue, dans lequel chaque barre de retenue est dimensionnée et configurée afin d'engager l'un des sites d'engagement sélectionné en vue de fournir une force de rétraction radiale associée.

7. Écarteur de plaies chirurgicales selon la revendication 6, dans lequel les sites d'engagement des éléments de retenue incluent une pluralité d'échelons, dans lesquels chacun des échelons est associé à une force de rétraction différente.

8. Écarteur de plaies chirurgicales selon la revendication 6, dans lequel :
les sites d'engagement incluent une pluralité de sillons, dans lesquels chaque sillon est associé à une force de rétraction différente ; et
chaque barre de retenue du second anneau inclut un levier de déviation afin d'engager l'un des sillons sélectionné pour fournir la force de rétraction associée.

9. Écarteur de plaies chirurgicales selon la revendication 6, dans lequel :
les sites d'engagement incluent une pluralité d'élargissements, dans lesquels chaque élargissement est associé à une force de rétraction différente ; et
chaque barre de retenue du second anneau a des propriétés pour engager l'un des élargissements sélectionné pour fournir la force de rétraction associée.
